(19) Europäisches Patentamt
European Patent Office
Office européen des brevets

(11) **EP 2 216 011 A2**

(12) **EUROPEAN PATENT APPLICATION**

(43) Date of publication:
**11.08.2010  Bulletin 2010/32**

(21) Application number: **10305127.2**

(22) Date of filing: **08.02.2010**

(51) Int Cl.:
*A61K 8/31* (2006.01)     *A61K 8/37* (2006.01)
*A61K 8/39* (2006.01)     *A61K 8/45* (2006.01)
*A61K 8/86* (2006.01)     *A61K 8/92* (2006.01)
*A61Q 5/12* (2006.01)     *A61K 8/36* (2006.01)
*A61Q 5/00* (2006.01)

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**
Designated Extension States:
**AL BA RS**

(30) Priority: **09.02.2009  US 150833 P
09.02.2009  US 150836 P
09.02.2009  US 150857 P
09.02.2009  US 150858 P**

(71) Applicant: **L'Oréal
75008 Paris (FR)**

(72) Inventors:
• **Nguyen, Nghi Van
Edison, NJ 08820 (US)**
• **Hashimoto, Sawa
Westfield, NJ 07090 (US)**
• **Cannell, David. W
New Hope, PA 18938 (US)**

(74) Representative: **Dossmann, Gérard
Casalonga & Partners
Bayerstrasse 71-73
80335 München (DE)**

(54) **Clear carrier compositions for lipophilic compounds, and method of treating keratinous substrates using such compositions**

(57)     The present invention relates to a composition comprising:

(a) at least one alkoxylated monoamine;

(b) at least one organic acid chosen from alkyl acids, alkoxylated monoacids, and mixtures thereof;

(c) at least one lipophilic compound; and

(d) at least one solvent comprising water.

Such a composition is clear in appearance, and stable.

The present invention also relates to a method of making such a clear composition, and to a method of cosmetic treatment of a keratinous substrate using such a composition.

**Description**

TECHNICAL FIELD

The present invention relates to carrier compositions comprising lipophilic ingredients. More particularly, the present invention provides for a clear composition containing one or more lipophilic ingredients, which remains clear even when diluted in particular with water.

The invention further relates to methods of treating keratinous substrates using such compositions.

BACKGROUND OF THE DISCLOSURE

Cosmetic and personal care products are available in various forms and one of the forms that are desired by many consumers is a clear aqueous product. At the same time, the consumer expects that such a product will provide desirable cosmetic benefits to keratinous substrates such as hair and skin.

These cosmetic benefits can be provided by the presence of water-insoluble ingredients, for example, oils, silicones and other lipophilic materials, in the product.

However, certain water-insoluble ingredients, which are oftentimes desirable for the treatment of keratinous substrates, are inherently difficult to incorporate into aqueous systems, such as shampoos, conditioners and skin care compositions, without forming a traditional emulsion in either cream or lotion form. Oftentimes, the presence of such ingredients at levels that would impart appreciable cosmetic benefits to hair or skin and/or properties to cosmetic and personal care products results in unstable formulations resulting in undesirable phase separations in aqueous systems.

Therefore, in the formulation of clear aqueous compositions, water-insoluble compounds do not lend themselves to being used therein, due to their inability to significantly associate with the water present in the system. As a result, the presence of these water-insoluble ingredients is generally minimal in personal care products and cosmetic products that employ aqueous systems. Thus, the difficulties in formulating such compositions deprives the consumer of products that can better deliver cosmetic benefits to hair and skin such as conditioning, cleansing, coloring of hair, styling of hair, skin care, and better application and spreadability of products.

Thus, there remains a need for an aqueous composition which can carry increased amounts of water-insoluble materials while remaining both homogeneous and clear in appearance. There also remains a need for an aqueous system which can carry increased amounts of water-insoluble materials such as oils and other lipophilic ingredients in order to deliver desirable benefits to hair and skin.

It has been surprisingly and unexpectedly discovered that the combination of at least one alkoxylated monoamine, at least one organic acid chosen from alkyl acids, alkoxylated monoacids and mixtures thereof, at least one solvent, and at least one water-insoluble compound such as a lipophilic compound, yields a composition which is clear in appearance and stable. Moreover, the inventive composition remains clear and stable even if additional solvent such as in particular water is added thereto.

It has also been discovered that the use of this clear composition on keratinous substrates, such as hair and skin, results in desirable and beneficial effects on the substrates, such as in particular improved delivery of active ingredients, improved cosmetic effects such as improved color retention on colored-treated hair, improved conditioning, improved hair styling effects and manageability, improved shine, improved protection from environmental and chemical damage, and enhanced color is the case of coloring compositions.

BRIEF SUMMARY OF THE DISCLOSURE

The present invention is directed to a composition comprising:

(a) at least one alkoxylated monoamine;
(b) at least one organic acid chosen from alkyl acids, alkoxylated monoacids, and mixtures thereof;
(c) at least one lipophilic compound ;and
(d) at least one solvent comprising water.
Such a composition is clear in appearance, and stable.

The present invention is also directed to a method of making a clear composition involving the steps of:

(a) providing at least one alkoxylated monoamine;
(b) providing at least one organic acid chosen from alkyl acids, alkoxylated monoacids, and mixtures thereof;
(c) providing at least one lipophilic compound;
(d) providing at least one solvent comprising water; and

(e) mixing the compounds as defined in steps (a) to (d) to form a composition that is clear in appearance.

The present invention is further directed to a method of cosmetic treatment of a keratinous substrate involving the step of applying onto said keratinous substrate a composition as defined herein.

DETAILED DESCRIPTION OF THE DISCLOSURE

The term "comprising" (and its grammatical variations) as used herein is used in the inclusive sense of "having" or "including" and not in the exclusive sense of "consisting only of". The terms "a" and "the" as used herein are understood to encompass the plural as well as the singular.

Other than in the operating examples, or where otherwise indicated, all numbers expressing quantities of ingredients and/or reaction conditions are to be understood as being modified in all instances by the term "about".

The term "lipophilic" means those compounds which are soluble in oils and either completely or partially insoluble in water. In accordance with the present invention, the lipophilic compounds preferably have a solubility in water at 25°C and at atmospheric pressure of less than 5% by weight, more preferably less than 1% by weight, even more preferably less than 0.5 % by weight and better still less than 0.1% by weight.

"At least one" as used herein means one or more and thus includes individual components as well as mixtures/combinations thereof.

The term "clear" as used herein means transparent such that a person is able to see through the composition with their naked eye. The term "clear" as used herein is not meant to encompass those compositions which a person cannot see through with their naked eye such as those which are pearlescent, frosted, hazy, opaque, or cloudy in appearance.

The clarity of the compositions of the present invention can be determined using the McFarland scale, which is based on the McFarland Equivalence Turbidity Standard Test (Remel, 12076 Santa Fe Drive, Lenexa, KS 66215, USA). Preferably, the compositions according to the present invention have a McFarland turbidity standard value, as visually determined, equal to or less than 0.5 on the McFarland scale.

The term "stable" as used herein means that the composition does not exhibit phase separation.

The term "carrier system for lipophilic compounds" means a system that delivers a lipophilic ingredient into an aqueous phase by incorporation or solubilization. The lipophilic carrier system of the present invention is capable of bringing lipophilic compounds into an aqueous phase such that the aqueous phase remains clear and stable.

"Substituted," as used herein, means comprising at least one substituent. Non-limiting examples of substituents include atoms, such as oxygen atoms and nitrogen atoms, as well as functional groups, such as hydroxyl groups, ether groups, alkoxy groups, acyloxyalkyl groups, oxyalkylene groups, polyoxyalkylene groups, carboxylic acid groups, amine groups, acylamino groups, amide groups, halogen containing groups, ester groups, thiol groups, sulphonate groups, thiosulphate groups, siloxane groups, and polysiloxane groups. The substituent(s) may be further substituted.

"Alkoxylated" as used herein means comprising at least one alkoxy group. As used herein, an alkoxy group is a group corresponding to the formula $-O-CHR-(CH_2)_n-$, wherein R represents H or a C1-C5 alkyl group, and wherein n is an integer ranging from 1 to 6.

ALKOXYLATED MONOAMINES

Non-limiting preferred examples of suitable alkoxylated monoamines include compounds corresponding to the formula (I):

$$RN[(R'CHCH_2O)_xH][(R'CHCH_2O)_yH] \qquad (I)$$

wherein R is a hydrocarbon radical containing at least 6 carbon atoms, preferably from 8 to 30 carbon atoms, more preferably from 12 to 24 carbon atoms. R can be linear or branched, acyclic or cyclic, saturated or unsaturated, aliphatic or aromatic, substituted or unsubstituted.

x and y, independently of one another, represent numbers of from 0 to 100 provided that the sum of x+y is greater than 0; the groups R', which may be identical or different, represent hydrogen, or an alkyl group such as in particular a methyl group. Preferably, the two R' groups are different: one R' group is hydrogen, and the other one is methyl.

Preferably, R is a linear or branched, acyclic alkyl or alkenyl group or an alkyl phenyl group; x and y, independently of one another, are each preferably a number from 0 to 30.

Examples of preferred alkoxylated monoamines for use in the present invention which correspond to formula (I) are PEG-2 cocamine, PEG-3 cocamine, PEG-5 cocamine, PEG-10 cocamine, PEG-15 cocamine, PEG-20 cocamine, PEG-2 lauramine, PEG-12 palmitamine, PEG-2 rapeseedamine, PEG-2 oleamine, PEG-5 oleamine, PEG-6 oleamine, PEG-10 oleamine, PEG-15 oleamine, PEG-20 oleamine, PEG-25 oleamine, and PEG-30 oleamine. Other examples are alkoxylated derivatives of soyamine, stearamine and tallow amine.

Further non-limiting examples of preferred alkoxylated monoamines include compounds corresponding to for-

mula (II):

$$RNR''[(R'CHCH_2O)_xH] \qquad (II)$$

wherein R is a hydrocarbon radical containing at least 6 carbon atoms, preferably from 8 to 30 carbon atoms, more preferably from 12 to 24 carbon atoms. R can be linear or branched, acyclic or cyclic, saturated or unsaturated, aliphatic or aromatic, substituted or unsubstituted.

x represents a number of from 1 to 100;

R' represents hydrogen, or an alkyl group such as in particular a methyl group; and

R'' is a hydrogen or a hydrocarbon radical.

Typically, R is a linear or branched, acyclic alkyl or alkenyl group or an alkyl phenyl group; x is preferably a number from 1 to 30.

When R'' in formula (II) is a hydrocarbon radical group, this group may be linear or branched, saturated or unsaturated, substituted or unsubstituted. The hydrocarbon radical represented by R'' may also contain an alkoxylated moiety (as defined by $[(R'CHCH_2O)_yH]$), and/or heteroatoms such as oxygen. When R'' contains at least one alkoxylated moiety, the total number of alkoxylation in the formula (II) may range from 1 to 120.

Additional non-limiting preferred examples of alkoxylated monoamines include compounds corresponding to formula (III):

$$R(R'CHCH_2O)_x(R'CHCH_2O)_yNH_2 \qquad (III)$$

wherein R is a hydrocarbon radical containing at least 6 carbon atoms, preferably from 8 to 30 carbon atoms, more preferably from 12 to 24 carbon atoms. R can be linear or branched, acyclic or cyclic, saturated or unsaturated, aliphatic or aromatic, substituted or unsubstituted.

x and y, independently of one another, represent numbers of from 0 to 100 with the proviso that the sum of x+y is greater than 0;

the groups R', which may be identical or different, represent hydrogen, or an alkyl group such as in particular a methyl group. Preferably, the two R' groups are different: one R' group is hydrogen, and the other one is methyl.

Typically, R is a linear or branched, acyclic alkyl or alkenyl group or an alkyl phenyl group; x and y, independently of one another, are each preferably a number from 0 to 30.

Examples of alkoxylated monoamines for use in the present invention which correspond to formula (III) are polyetheramines containing a monoamine group. These polyetheramines are commercially available from Hunstman under the tradename Jeffamine (M series such as M-600, M-1000, M-2005 and M-2070) and Surfonamine series (B-60, B-100, B-200, L-100, L-200, L-207, L-300).

The at least one alkoxylated monoamine can be present in the composition of the present invention in an amount of from 0.1 to 50 % by weight, preferably from 0.5 to 30% by weight, more preferably from 1 to 20% by weight, based on the total weight of the composition.

ALKYL ACIDS

The at least one alkyl acid of the composition of the present invention is preferably chosen from fatty monocarboxylic acids, fatty phosphoric acids and mixtures thereof.

By "fatty", it is meant that such acids contain a fatty chain having at least 6 carbon atoms, preferably from 8 to 30 carbon atoms.

Non-limiting preferred examples of fatty monocarboxylic acids include fatty acids corresponding formula (IA):

$$RCOOH \qquad (IA)$$

wherein R is a hydrocarbon radical containing at least 6 carbon atoms, preferably from 8 to 30 carbon atoms, more preferably from 12 to 24 carbon atoms. R can be linear or branched, acyclic or cyclic, saturated or unsaturated, aliphatic or aromatic, substituted or unsubstituted. Typically, R is a linear or branched, acyclic alkyl or alkenyl group or an alkyl phenyl group.

Suitable fatty acids include, but are not limited to the following examples: arachidic acid, arachidonic acid, beeswax acid, capric acid, caproic acid, caprylic acid, coconut acid, isostearic acid, lauric acid, linoleic acid, linolenic acid, myristic acid, oleic acid, olive acid, palmitic acid, rapeseed acid, stearic acid, behenic acid, tallow acid, undecanoic acid, undecylenic acid, wheat germ acid and mixtures thereof.

Preferred fatty acids in the present invention include linoleic acid, oleic acid, isostearic acid, stearic acid and mixtures thereof.

Non-limiting preferred examples of fatty phosphoric acids include compounds corresponding to Formula (IIA):

$$R\text{-}O\text{-}P(O)(OH)_2 \qquad (IIA)$$

wherein R is a hydrocarbon radical containing at least 6 carbon atoms, preferably from 8 to 30 carbon atoms, more preferably from 12 to 24 carbon atoms. R can be linear or branched, acyclic or cyclic, saturated or unsaturated, aliphatic or aromatic, substituted or unsubstituted. Typically, R is a linear or branched, acyclic alkyl or alkenyl group or an alkyl phenyl group.

Typical suitable fatty phosphoric acids include capryl phosphate, caprylyl phosphate, lauryl phosphate, oleyl phosphate, isostearyl phosphate, stearyl phosphate, cetyl phosphate and mixtures thereof.

ALKOXYLATED MONOACIDS

Non-limiting preferred examples of alkoxylated monoacids include compounds corresponding to formula (IB):

$$RO[CH_2O]_u[(CH_2)_xCH(R')(CH_2)_yO]_v[CH_2CH_2O]_w[CH_2COOH \qquad (IB)$$

wherein:

R is a hydrocarbon radical containing from 6 to 40 carbon atoms, preferably from 8 to 30 carbon atoms, more preferably from 12 to 24 carbon atoms;
u, v and w, independently of one another, represent numbers of from 0 to 60, provided that the sum u + v + w is greater than 0;
x and y, independently of one another, represent numbers of from 0 to 13, where the sum x + y is greater than or equal to 0;
R' represents hydrogen or an alkyl group, and preferably a methyl group.

Compounds corresponding to formula (IB) can be obtained by alkoxylation of alcohols of formula ROH - with R as defined in formula (IB) - with ethylene oxide as the sole alkoxide or with several alkoxides and subsequent oxidation. The numbers u, v, and w each represent the degree of alkoxylation. Whereas, on a molecular level, the numbers u, v and w and the total degree of alkoxylation can only be integers, including zero, on a macroscopic level they are mean values in the form of broken numbers.

In formula (IB), R is linear or branched, acyclic or cyclic, saturated or unsaturated, aliphatic or aromatic, substituted or unsubstituted. Typically, R is a linear or branched, acyclic $C_{6-40}$ alkyl or alkenyl group or a $C_{1-40}$ alkyl phenyl group, more typically a $C_{8-22}$ alkyl or alkenyl group or a $C_{4-18}$ alkyl phenyl group, and even more typically a $C_{12-18}$ alkyl group or alkenyl group or a $C_{6-16}$ alkyl phenyl group;
the sum u+v+w, is preferably a number ranging from 2 to 20, more preferably a number ranging from 3 to 17 and most preferably a number from 5 to 15;
the sum x+y is typically a number from 1 to 13, more preferably a number from 1 to 10.

Suitable alkoxylated monoacids of the present invention include, but are not limited to, the following representatives referred to by their INCI names (INCI: nomenclature for raw materials according to the International Cosmetic Ingredient Dictionary, 7th Edition, published by the Cosmetic, Toiletry and Fragrance Association Inc. (CTFA), Washington D.C., USA): Butoxynol-5 Carboxylic Acid, Butoxynol-19 Carboxylic Acid, Capryleth-4 Carboxylic Acid, Capryleth-6 Carboxylic Acid, Capryleth-9 Carboxylic Acid, Ceteareth-25 Carboxylic Acid, Coceth-7 Carboxylic Acid, $C_{9-11}$ Pareth-6 Carboxylic Acid, $C_{11-15}$ Pareth-7 Carboxylic Acid, $C_{12-13}$ Pareth-5 Carboxylic Acid, $C_{12-13}$ Pareth-8 Carboxylic Acid, $C_{12-13}$ Pareth-12 Carboxylic Acid, $C_{12-15}$ Pareth-7 Carboxylic Acid, $C_{12-15}$ Pareth-8 Carboxylic Acid, $C_{14-15}$ Pareth-8 Carboxylic Acid, Deceth-7 Carboxylic Acid, Laureth-3 Carboxylic Acid, Laureth-4 Carboxylic Acid, Laureth-5 Carboxylic Acid, Laureth-6 Carboxylic Acid, Laureth-8 Carboxylic Acid, Laureth-10 Carboxylic Acid, Laureth-11 Carboxylic Acid, Laureth-12 Carboxylic Acid, Laureth-13 Carboxylic Acid, Laureth-14 Carboxylic Acid, Laureth-17 Carboxylic Acid, PPG-6-Laureth-6 Carboxylic Acid, PPG-8-Steareth-7 Carboxylic Acid, Myreth-3 Carboxylic Acid, Myreth-5 Carboxylic Acid, Nonoxynol-5 Carboxylic Acid, Nonoxynol-8 Carboxylic Acid, Nonoxynol-10 Carboxylic Acid, Octeth-3 Carboxylic Acid, Octoxynol-20 Carboxylic Acid, Oleth-3 Carboxylic Acid, Oleth-6 Carboxylic Acid, Oleth-10 Carboxylic Acid, PPG-3-Deceth-2 Carboxylic Acid, Capryleth-2 Carboxylic Acid, Ceteth-13 Carboxylic Acid, Deceth-2 Carboxylic Acid, Hexeth-4 Carboxylic Acid, Isosteareth-6 Carboxylic Acid, Isosteareth-11 Carboxylic Acid, Trudeceth-3 Carboxylic Acid, Trideceth-6 Carboxylic Acid, Trideceth-8 Carboxylic Acid, Trideceth-12 Carboxylic Acid, Trideceth-3 Carboxylic Acid, Trideceth-4 Carboxylic Acid, Trideceth-7 Carboxylic Acid, Trideceth-15 Carboxylic Acid, Trideceth-19 Carboxylic Acid, Undeceth-5 Carboxylic Acid and mixtures thereof.

The at least one organic acid chosen from alkyl acids, alkoxylated monoacids and mixtures thereof is preferably present in the composition in an amount of from 0.1 to 50% by weight, preferably from 0.5 to 30% by weight, and more preferably 1 to 20% by weight, based on the total weight of the composition.

Preferably, the ratio of the amine number of the at least one alkoxylated monoamine to the total acid number of organic acid(s) chosen from alkyl acids, alkoxylated monoacids and mixtures thereof, is from 1:10 to 10:1, more preferably from 1:5 to 5:1, and even more preferably from 1:2 to 2:1. Also, the composition remains clear when diluted with any ratio or amount of additional solvent.

Acid and amine numbers are generally determined by acid-base titration in the presence of a color indicator based on the European and American Pharmacopoeias and Standard ISO 660.

LIPOPHILIC COMPOUND

The at least one lipophilic compound may, for example, be chosen from oils, fatty esters, hydrocarbon oils, silicones, waxes, fatty alcohols, lipophilic vitamins and esters thereof, organic sunscreens, phospholipids, and mixtures thereof.

Non-limiting examples of oils include plant oils such as olive oil, avocado oil, coconut oil, safflower oil, almond oil, castor oil, jojoba oil, peanut oil, sesame oil, hazelnut oil, sunflower oil, apricot kernel oil, grapeseed oil, linseed oil and palm oil.

Non-limiting examples of hydrocarbon oils include mineral oil, petrolatum, and $C_{10}$-$C_{40}$ hydrocarbons which may be aliphatic (with a straight, branched or cyclic chain), aromatic, arylaliphatic such as paraffins, iso-paraffins, isododecanes, aromatic hydrocarbons, and mixtures thereof.

Non-limiting examples of silicones include phenyltrimethicone, dimethicone, cyclomethicone, dimethicone copolyol, aminosilicone, laurylmethicone copolyol, cetyl dimethicone, cetyl triethylammonium dimethicone copolyol phthalate, dimethicone copolyol lactate, silicone quaternium 13, stearalkonium dimethicone copolyol phthalate, stearaminopropyl dimethicone and polyorganosiloxanes such as polydimethylsiloxane.

Non-limiting examples of waxes include paraffin wax, beeswax, candelilla wax, carnauba wax, jasmine wax, jojoba wax and mimosa wax.

Non-limiting preferred examples of fatty alcohols include compounds of formula:

R-OH

where R represents a hydrocarbon radical containing at least three carbon atoms, preferably from 8 to 30, more preferably from 12 to 24 carbon atoms, and which may be linear or branched, acyclic or cyclic, saturated or unsaturated, aliphatic or aromatic, substituted or unsubstituted. Typically, R is a linear or branched, acyclic alkyl or alkenyl group or an alkyl phenyl group.

Non-limiting preferred fatty esters include esters formed from $C_{8-30}$ fatty acids and $C_{1-10}$ alcohols and esters formed from the fatty alcohols as defined hereabove and $C_{1-10}$ carboxylic acids.

According to a preferred embodiment, the at least one lipophilic compound is chosen from isopropyl palmitate, capric/caprylic triglyceride, isodecyl neopentanoate, polylsobutylene, phloretin, ellagic acid, vitamin D, vitamin E, vitamin E acetate, vitamin A, vitamin A palmitate, 2-oleamido-1,3-octadecanediol, octyl methoxycinnamate, octyl salicylate, 18-methyleicosanoic acid, and mixtures thereof.

The at least one lipophilic compound is present in the composition in a preferred amount of from 0.1 to 50% by weight, more preferably from 0.1 to 30% by weight, and even more preferably from 0.5 to 15% by weight, based on the total weight of the composition.

SOLVENT

The solvent is typically present in an amount from 10 to 95% by weight, preferably from 50 to 85% by weight, and more preferably from 60 to 80% by weight, based on the total weight of the composition. The solvent comprises water such as deionized water, alone or in combination with at least one $C_1$-$C_4$ alcohol. Alcohols include ethanol, propanol and butanol. Preferably, the alcohol is chosen from ethanol, isopropanol and mixtures thereof.

The solvent preferably comprises at least 20% by weight of water, more preferably at least 50% by weight, even more preferably at least 80% by weight, based on the total weight of the solvent.

AUXILIARY INGREDIENTS

The composition may optionally contain at least one auxiliary ingredient. The auxiliary ingredients may include in particular film forming agents, proteins, amino acids, cationic conditioners, cationic polymers, nonionic surfactants, anionic surfactants, amphoteric surfactants, zwitterionic surfactants, viscosity modifiers, antibacterial agents, sunscreens, preservatives, pH adjusting agents, bleaching agents, hair dyeing agents, perfumes, sequestering agents, antidandruff agents, alpha or beta hydroxy acids or alpha ketoacids, and mixtures thereof.

Non-limiting examples of film forming agents can be chosen from anionic compounds, non-ionic compounds, amphoteric compounds, zwitterionic compounds, proteins, viscosity modifiers, cationic polymers, polyamides, polyaminoamides, polyesters, silicone resins, polysaccharides, silicone fluids, polyacrylamides, starches, gums and mixtures thereof.

Non-limiting examples of proteins include collagen, deoxyribonuclease, iodized corn protein, milk protein, protease, serum protein, silk, sweet almond protein, wheat germ protein, wheat protein, alpha and beta helix of keratin proteins, hair proteins, such as intermediate filament proteins, high-sulfur proteins, ultrahigh-sulfur proteins, intermediate filament-associated proteins, high-tyrosine proteins, high-glycine tyrosine proteins, tricohyalin, and mixtures thereof.

Non-limiting examples of amino acids include amino acids derived from the hydrolysis of various proteins as well as the salts, esters, and acyl derivatives thereof. Non-limiting examples of such amino acid agents include amphoteric amino acids such as alkylamido alkylamines, i.e. stearyl acetyl glutamate, capryloyl silk amino acid, capryloyl collagen amino acids, capryloyl keratin amino acids, capryloyl pea amino acids, cocodimonium hydroxypropyl silk amino acids, corn gluten amino acids, cysteine, glutamic acid, glycine, hair keratin amino acids, amino acids such as asparatic acid, threonine, serine, glutamic acid, proline, glycine, alanine, cystine, valine, methionine, isoleucine, leucine, tyrosine, phenylalanine, cysteic acid, lysine, histidine, arginine, cysteine, tryptophan, citrulline, lysine, silk amino acids, wheat amino acids and mixtures thereof.

Non-limiting examples of cationic conditioners include quaternium-27, behenamidopropyl PG-dimonium chloride, hydroxyethyl tallowdimonium chloride, stearalkonium chloride and cetrimonium chloride.

Non-limiting examples of cationic polymers include hexadimethrine chloride, polyquaternium-4, polyquaternium-6, polyquaternium-7, polyquaternium-10, polyquaternium-11, polyquaternium-16, polyquaternium-22 and polyquaternium-32.

Non-limiting examples of nonionic surfactants includes alkoxylated derivatives of the following: fatty alcohols, alkyl phenols, fatty acid esters and fatty acid amides,
wherein the alkyl chain is in the $C_{12-50}$ range, typically in the $C_{16-40}$ range, more typically in the $C_{24}$ to $C_{40}$ range, and having from 1 to 110 alkoxy groups. The alkoxy groups are selected from the group consisting of $C_2$-$C_6$ oxides and their mixtures, with ethylene oxide, propylene oxide, and their mixtures being the typical alkoxides. The alkyl chain may be linear, branched, saturated, or unsaturated. Of these alkoxylated non-ionic surfactants, the alkoxylated fatty alcohols are preferred, and the ethoxylated alcohols and propoxylated alcohols are more preferred. The alkoxylated alcohols may be used alone or in mixtures with those alkoxylated materials disclosed herein-above.

Representative preferred examples of such ethoxylated fatty alcohols include laureth-3 (a lauryl ethoxylate having an average degree of ethoxylation of 3), laureth-23 (a lauryl ethoxylate having an average degree of ethoxylation of 23), ceteth-10 (a cetyl alcohol ethoxylate having an average degree of ethoxylation of 10), steareth-10 (a stearyl alcohol ethoxylate having an average degree of ethoxylation of 10), steareth-2 (a stearyl alcohol ethoxylate having an average degree of ethoxylation of 2), steareth-100 (a stearyl alcohol ethoxylate having an average degree of ethoxylation of 100), beheneth-5 (a behenyl alcohol ethoxylate having an average degree of ethoxylation of 5), beheneth-10 (a behenyl alcohol ethoxylate having an average degree of ethoxylation of 10), and mixtures of the precedings.

Commercially available corresponding nonionic surfactants are for example Brij® nonionic surfactants from Croda, Inc., Edison, NJ. Typically, Brij® is the condensation products of aliphatic alcohols with from 1 to 54 moles of ethylene oxide, the alkyl chain of the alcohol being typically a linear chain and having from 8 to 22 carbon atoms, for example, Brij 72 (i.e., Stear...-2) and Brij 76 (i.e., Steareth-10).

Also useful herein as nonionic surfactants are alkyl glycosides, which are the condensation products of long chain alcohols, e.g. $C_8$-$C_{30}$ alcohols, with sugar or starch polymers. These compounds can be represented by the formula (S)n-O-R
wherein S is a sugar moiety such as glucose, fructose, mannose, galactose, and the like; n is an integer of from 1 to 1000, and R is a $C_8$-$C_{30}$ alkyl group. Examples of long chain alcohols from which the alkyl group can be derived include decyl alcohol, cetyl alcohol, stearyl alcohol, lauryl alcohol, myristyl alcohol, oleyl alcohol, and the like. Preferred examples of these surfactants are alkyl polyglucosides wherein S is a glucose moiety, R is a $C_8$-$C_{20}$ alkyl group, and n is an integer of from 1 to 9. Commercially available examples of these surfactants include decyl polyglucoside (available as APG® 325 CS) and lauryl polyglucoside (available as APG® 600CS and 625 CS), all the above-identified polyglucosides APG® are available from Cognis, Ambler, Pa. Also useful herein are sucrose ester surfactants such as sucrose cocoate and sucrose laurate.

Other nonionic surfactants suitable for use in the present invention are glyceryl esters and polyglyceryl esters, including but not limited to, glyceryl monoesters, typically glyceryl monoesters of $C_{16}$-$C_{22}$ saturated, unsaturated and branched chain fatty acids such as glyceryl oleate, glyceryl monostearate, glyceryl monoisostearate, glyceryl monopalmitate, glyceryl monobehenate, and mixtures thereof, and polyglyceryl esters of $C_{16}$-$C_{22}$ saturated, unsaturated and branched chain fatty acids, such as polyglyceryl-4 isostearate, polyglyceryl-3 oleate, polyglyceryl-2 sesquioleate, triglyceryl diisostearate, diglyceryl monooleate, tetraglyceryl monooleate, and mixtures thereof.

Also useful herein as nonionic surfactants are sorbitan esters. Preferable are sorbitan esters of $C_{16}$-$C_{22}$ fatty acids. Because of the manner in which they are typically manufactured, these sorbitan esters usually comprise mixtures of mono-, di-, tri-, etc. esters. Representative examples of suitable sorbitan esters include sorbitan monooleate (e.g., SPAN® 80), sorbitan sesquioleate (e.g., Arlacel® 83 from Croda, Inc., Edison, NJ), sorbitan monoisostearate (e.g., CRILL® 6 from Croda, Inc., Edison, N.J.), sorbitan stearates (e.g., SPAN® 60), sorbitan trioleate (e.g., SPAN® 85), sorbitan tristearate (e.g., SPAN® 65), sorbitan dipalmitates (e.g., SPAN® 40), and sorbitan isostearate. Sorbitan monoisostearate and sorbitan sesquioleate are particularly preferred emulsifiers for use in the present invention.

Also suitable for use as nonionic surfactants are alkoxylated derivatives of glyceryl esters, sorbitan esters, and alkyl polyglycosides, wherein the alkoxy groups is selected from the group consisting of $C_2$-$C_6$ oxides and their mixtures, with ethoxylated or propoxylated derivatives of these materials being typical. Nonlimiting examples of commercially available ethoxylated materials include ethoxylated sorbitan mono-, di- and/or tri-esters of $C_{12}$ to $C_{18}$ fatty acids with an average degree of ethoxylation of from 2 to 20, such as the products sold under the name TWEEN® by the company Uniqema.

Non-limiting examples of anionic surfactants include compounds in the classes known as alkyl sulfates, alkyl ether sulfates, alkyl sulfonates, alkyl ether sulfonates, sulfate esters of an alkylphenoxy polyoxyethylene ethanol, alphaolefin sulfonates, beta alkyloxy alkene sulfonates, alkyl arylsulfonates, alkyl carbonates, succinamates, sulfosuccinates, sarcosinates, octoxynol or nonoxynol phosphates, taurates, fatty taurides, sulfated monoglycerides, fatty acid amino polyoxyethylene sulfates, isethionates and mixtures thereof. Specific examples of anionic surfactants include the ammonium, monoethanolamine, diethanolamine, triethanolamine, isopropylamine, sodium, potassium, lithium, or magnesium salts of lauryl sulfate, dodecylbenzenesulfonate, lauryl sulfosuccinate, lauryl ether sulfate, lauryl sarcosinate, cocomethyl tauride, and sulfosuccinate half ester amide and mixtures thereof.

Non-limiting examples of amphoteric and zwitterionic surfactants include alkyl, alkyl dimethyl, alkylamido, alkyl amide, alkylamidopropyl, or alkyl dimethylammonium betaine; alky amidopropyl or alkyl sulfobetaine; alkyl, alkylampho, or alkylamphocarboxy glycinate; alkyl, or alkyl substituted imidazoline mono or dicarboxylate; sodium salts of alkyl mono- or dicarboxylates; alkyl beta amino acids; alkyl amidopropyl, or alkyl ether hydroxysultaine; alkyl amidopropyl dimethyl ammonia acetate; alkyl ampho mono-or diacetate; alkyl, or alkyl ampho, or alkyl imino dipropionate; alkyl amphopropionate; alkyl beta amino propionic acid; alkyl dipropionate; alkyl beta iminodipropionate; branched or n-alkyl dimethylamidopropionate; alkyl carboxylated propionate; alkyl, or methyl alkyl imidazoline; fluorinated alkyl amphoteric mixtures.

Specific examples include cocamidopropyl betaine, lauramidopropyl betaine, coco/oleamidopropyl betaine, coco betaine, oleyl betaine, cocamidopropyl hydroxysultaine, tallowamidopropyl hydroxysultaine and dihydroxyethyl tallow glycinate and mixtures thereof.

Non-limiting examples of viscosity modifiers include water swellable/soluble cationic polymers from quaternized polysaccharides such as trimethyl ammonium substituted epoxide of hydroxyethyl cellulose, diallyldimethyl ammonium salts of hydroxyethylcellulose, deacylated chitin or chitosan, dihydroxypropyl chitosan trimonium chloride, hydroxyproplptrimethyl ammonium chloride guar, locust bean, or konjac mannan gum; quaternized synthetics such as acrylamide dimethyl diallyl ammonium chloride copolymers, acrylamide/dimethyl diallyl ammonium chloride/acrylic acid terpolymer, quaternized poly (vinyl pyrrolidone/dimethyl amino ethylmethacrylate), poly (vinylpyrrolidone/methacrylamidopropyl trimethylammonium chloride), polyvinyl pyrrolidone/methylvinylimidazolinium chloride or methyl sulfate copolymer, chloroethylether/dimethylaminopropylamine/adipate or azelate terpolymer, vinylpyrrolidone/methacrylamidopropyl trimethylammonium chloride, acrylonitrile/acrylic acid/dimethylpropanediammonium acrylates sulfate terpolymer.

Further suitable viscosity modifiers include anionic or nonionic polysaccharide polymers such as gum tragacanth, sodium or propylene glycol alginate, kappa-, iota-,or lambda-carrageenan, guar or hydroxyl propyl guar gum, karaya gum, gum arabic, locust bean gum, konjac mannan gum, gellan, xanthan, succinoglycan or its acidic or enzymatic hydrolysates, sodium carboxymethyl cellulose, methycellulose, hydroxylethylcellulose, hydroxypropylmethylcellulose, and hydroxypropylcellulose; and/or hydrophobically modified anionic, cationic, or nonionic polymers such as, but not limited to, alkyl and/or substituted hydroxyethylcellulose, lauryl dimethyl ammonium substituted epoxide of hydroxyethylcellulose, propoxylated cellulosic, xanthan, succinoglycan, or polygalactomannoses, alkyl methacrylates/crosslinked acrylic acid copolymers and/or acrylonitrile/acrylates block copolymers.

Non-limiting examples of antibacterial agents include bacitracin, phenol, benzethonium chloride, erythromycin, neomycin, tetracycline, chlortetracycline and mixtures thereof.

Non-limiting examples of sunscreens include benzophenones, bornelone, butyl PABA, cinnamidopropyl trimethyl ammonium chloride, disodium distryrylbiphenyl disulfonate, PABA, potassium methoxycinnamate, butyl methoxydibenzoylmethane, octyl methoxycinnamate, oxybenzone, octocrylene, octyl salicylate, phenylbenzimidazole sulfonic acid, ethyl hydroxypropyl aminobenzoate, menthyl anthranilate, aminobenzoic acid, cinoxate, diethanolamine methoxycinnamate, glyceryl aminobenzoate, titanium dioxide, zinc oxide, oxybenzone, ethylhexyl dimethyl PABA, red petrolatum, and mixtures thereof.

Non-limiting examples of preservatives include polyvinyl alcohol, phenoxyethanol, benzyl alcohol, methyl paraben, propyl paraben and mixtures thereof.

Non-limiting examples of pH adjusting agents include potassium acetate, sodium carbonate, sodium hydroxide, phosphoric acid, succinic acid, sodium citrate, citric acid, boric acid, lactic acid, sodium hydrogen carbonate and mixtures thereof.

Bleaching agents include, but are not limited to, hydrogen peroxide, perborate and persufate salts. EDTA and other aminocarboxylates may be used as sequestering agents.

Anti-dandruff agents such as zinc pyrithione, salicylic acid, climbazole, ketoconazole, sulfur piroctone olamine, selenium sulfide and mixtures thereof may also be used as an auxiliary ingredient.

The alpha hydroxy acids may exist in the keto acid form, or the ester form. Examples of such alpha hydroxy acids include glycolic acid, malic acid, pyruvic acid, mandelic acid, lactic acid, methyllactic acid, and mixtures thereof.

Also beta hydroxy acids such as salicylic acid, and derivatives thereof may be included in the compositions of the present invention. In addition, mixtures of the above alpha and beta hydroxyl acids or alpha ketoacids can be advantageously included.

The compositions described above are useful as compositions for treating keratinous substrates. These compositions include hair care products such as shampoos and conditioners, products for treating skin such as skin cleansers and personal hygiene products and products for cleaning and treating lips and nails.

For example, when the keratinous substrate being treated is hair, the compositions of the invention may impart shine, conditioning, color retention in particular when the compositions are formulated into a rinse-off product. In this case, the method of the present invention will include a rinsing step usually performed with water, after a leave-on time of the composition of at least 30 seconds.

Similar properties, along with styling, may be provided when the composition is in the form of a leave-on product.

When the keratinous substrate is skin, the compositions may impart protection from the sun (sunscreens) or provide skin benefits by serving as a carrier vehicle for skin actives (anti-acne, anti-wrinkle, etc.).

The method of treatment to be provided will depend on the keratinous substrate being targeted and, consequently, the specific ingredients contained in the composition used to effectuate the treatment. One of ordinary skill in the art will easily be able to determine these variables. Regardless of the type of treatment and/or the type of keratinous substrate chosen, the method of treatment will be performed by a composition which is clear in appearance, regardless of the degree of dilution.

The following examples are for illustrative purposes only and are not intended to limit the scope of the claims.

Example 1:

Various compositions (all of them qs to 100% with water), each containing a lipophilic compound, were prepared using the ingredients listed in Table 1 below, in which all quantities as expressed as percentages by weight with regard to the total weight of the composition.

These compositions were characterized as either clear or opaque using the McFarland scale. The McFarland scale is based on the McFarland Equivalence Turbidity Standard Test (Remel, 12076 Santa Fe Drive, Lenexa, KS 66215, USA). McFarland standards are used most commonly in microbiology as a reference to measure turbidity of bacterial suspensions in test tubes. The standards are generally prepared suspensions of either barium chloride or latex that range from a scale of 0.5 to 4. The higher the number, the more turbid the suspension. The latex suspension standard was used in this study.

Each composition in this study was placed into a clear glass test tube and was visually compared to the McFarland standards against a white card with contrasting black lines. A composition that did not exhibit phase separation, but visually appeared to possess a McFarland turbidity standard value of greater than 0.5 (> 0.5) on the McFarland scale was deemed to be opaque. Conversely, a composition that did not exhibit phase separation, but visually appeared to possess a McFarland turbidity standard value equal to, or less than 0.5 (≤ 0.5) on the McFarland scale was deemed to be clear.

Table 1:

|  | Organic acid | Alkoxylated monoamine | Lipophilic compound | Properties |
|---|---|---|---|---|
| 1a. | isostearic acid, 1.65% | PEG-15 cocamine, 5% | isodecyl neopentanoate, 1.0% | ≤0.5 (clear+ homogeneous) |
| 1b. | isostearic acid, 1.65% | - | isodecyl neopentanoate, 1.0% | Phases separated |
| 1c. | - | PEG-15 cocamine, 5% | isodecyl neopentanoate, 1.0% | >0.5 (opaque) |
| 2a. | isostearic acid, 1.65% | PEG-15 cocamine, 5% | mineral oil, 1.0% | ≤0.5 (clear+ homogeneous) |
| 2b. | isostearic acid, 1.65% | - | mineral oil, 1.0% | Phases separated |
| 2c. | - | PEG-15 cocamine, 5% | mineral oil, 1.0% | Phases separated |
| 3a. | isostearic acid, 1.65% | PEG-15 cocamine, 5% | isopropyl myristate, 1% | ≤0.5 (clear+ homogeneous) |
| 3b. | isostearic acid, 1.65% | - | isopropyl myristate, 1% | Phases separated |
| 3c. | - | PEG-15 cocamine, 5% | isopropyl myristate, 1% | >0.5 (opaque) |

The results above show that the compositions having either the alkoxylated monoamine or the organic acid exhibited phases separation or appeared opaque (> 0.5 rating on the McFarland scale). In contrast, the compositions having both the alkoxylated monoamine and the organic acid did not exhibit phases separation and were clear (≤ 0.5 rating on the McFarland scale).

Example 2:

Various compositions (all of them qs to 100% with water), each containing a lipophilic compound, were prepared using the ingredients listed in Table 2 below, in which all quantities as expressed as percentages by weight with regard to the total weight of the composition.

These compositions were characterized as either clear or opaque using the McFarland scale, using the same procedure as in example 1 above.

Table 2:

|  | Organic acid | Alkoxylated monoamine | Lipophilic compound | Properties |
|---|---|---|---|---|
| 1a. | laureth-5 carboxylic acid, 4.5% | PEG-15 cocamine, 3% | mineral oil, 1% | ≤0.5 (clear+ homogeneous) |
| 1b. | laureth-5 carboxylic acid, 4.5% | - | mineral oil, 1% | Phases separated |
| 1c. | - | PEG-15 cocamine, 3% | mineral oil, 1% | >0.5 (hazy, opaque) |
| 2a. | laureth-5 carboxylic acid, 4.5% | PEG-2 oleamine, 3.5% | mineral oil, 1% | ≤0.5 (clear+ homogeneous) |
| 2b. | laureth-5 carboxylic acid, 4.5% | - | mineral oil, 1.0% | Phases separated |
| 2c. | - | PEG-2 oleamine, 3.5% | mineral oil, 1% | Phases separated |
| 3a. | laureth-11 carboxylic acid, 3.6% | PEG-2 oleamine, 2.2% | coconut oil, 1% | ≤0.5 (clear+ homogeneous) |
| 3b. | laureth-11 carboxylic acid, 3.6% | - | coconut oil, 1% | >0.5 (opaque) |
| 3c. | - | PEG-2 oleamine, 2.2% | coconut oil, 1% | Phases separated |
| 4a. | laureth-11 carboxylic acid, 3.6% | PEG-15 cocamine, 2% | phloretin, 1% | ≤0.5 (clear+ homogeneous) |
| 4b. | laureth-11 carboxylic acid, 3.6% | - | phloretin, 1% | Phases separated |
| 4c. | - | PEG-15 cocamine, 2% | phloretin, 1% | Phases separated |

The results above show that the compositions having either the alkoxylated monoamine or the organic acid exhibited phases separation or appeared cloudy (> 0.5 rating on the McFarland scale). In contrast, the compositions having both the alkoxylated monoamine and the organic acid did not exhibit phases separation and were clear (≤ 0.5 rating on the McFarland scale).

Example 3: cosmetic benefits

Normal virgin hair (chemically untreated hair) was treated with the following compositions (all quantities being expressed as percentages by weight):

Composition 1: Clear composition (≤0.5 McFarland Scale): laureth-11 carboxylic acid (3.6%), PEG-2 oleamine (2%), vitamin A (retinyl palmitate) (1%), water (Q.S. to 100%).
Composition 2: Cloudy composition (> 0.5 McFarland Scale): laureth-11 carboxylic acid (3.6%), vitamin A (retinyl palmitate) (1%), water (Q.S. to 100%).
Composition 3: Separated composition: PEG-2 oleamine (2%), vitamin A (retinyl palmitate) (1%), and (Q.S. to 100%)).
Composition 4: Separated composition: vitamin A (retinyl palmitate) 1%, and water (Q.S. to 100%)

For each composition above, three 3g of natural level 6 hair swatches were treated with 100g of the composition for 3 hours at room temperature, and then rinsed with water for 1 minute.

The swatches were dried completely, then 1g of the hair was placed into 15g of ethanol overnight with gentle shaking.

The amount of vitamin A (retinyl palmitate) extracted from the hair was measured by the UV-Vis absorbance of the ethanol solution at 325 nm.

The following results were obtained:

| Composition | mg of Vit A/g hair |
|-------------|--------------------|
| 1 | 0.83 |
| 2 | 0.24 |
| 3 | 0.48 |
| 4 | 0.00 |

These data indicate that hair treated with composition 1 has the highest amount of vitamin A (retinyl palmitate) which is statistically significant from all other treatments. Therefore, the presently claimed composition delivers Vitamin A (retinyl palmitate) to hair, significantly better than any other compositions lacking one or more of its components.

Example 4: color retention on artificially colored hair

Bleached hair swatches colored with a commercial hair color product (Redken HiFusion R, 20 vol, 30 min at room temperature) were treated with the compositions of the present invention, then shampooed multiple times as indicated below.

Artificially colored hair swatches were treated for 5 minutes (0.4 g of composition for 1 g of hair) with the following compositions (all quantities being expressed as percentages by weight, using 5 swatches per composition:

Composition 1 (clear composition): laureth-5 carboxylic acid 5%, PEG-15 cocamine 3%, mineral oil 1%, water qs to 100%.
Composition 2 (clear composition): laureth-11 carboxylic acid 4%, PEG-2 oleamine 2.15%, coconut oil 1%, water qs to 100%.
Composition 3 (clear composition): laureth-5 carboxylic acid 5%, PEG-2 oleamine 3.56%, mineral oil 1%, water qs to 100%. Control: no treatment (shampoo only).

The treated hair swatches were then shampooed (using an aqueous solution of 15% by weight of sodium laureth-2 sulfate, pH 6, 0.4 g of shampoo / g of hair) for 15 seconds and rinsed with water for 10 seconds. The treatment and shampooing cycle was repeated up to 5 times.

The color L*a*b* values were measured before the treatment and after each shampooing cycle. Hair swatches that had no treatment were used as controls.

The color loss after 3 and 5 shampooing cycles was determined by colorimetric measurements. The color intensity of each hair swatch was determined using the CIELAB L*a*b* system using a Sphere Spectrophotometer SP60 Series. The color L*a*b* values were measured before the treatment, and after the treatment and shampooing cycles. The color intensity of each hair swatch was determined using the CIELAB L*a*b* system using a Konica Minolta Spectrophotometer 2600-D Series. Six measurements were taken on each swatch, three on each side of the swatch, at the top, middle and bottom of each side. The overall color change, represented by the $\Delta E$ value, is computed using the equation:

$$\Delta E = \sqrt{\left(L*_1 - L*_0\right)^2 + \left(a*_1 - a*_0\right)^2 + \left(b*_1 - b*_0\right)^2}$$

where $L*_0$, $a*_0$, and $b*_0$ are coordinates associated with initial color measurements right after coloring the hair and $L*_1$, $a*_1$, and $b*_1$ are coordinates associated with the final color measurements right after the 3$^{rd}$ and the 5$^{th}$ shampooing cycles. The L coordinate represents the color intensity of the swatch being tested based on a dark to light scale. The a coordinate represents the color position between red and green. The b coordinate represents the color position between yellow and blue. A higher $\Delta E$ value is representative of a greater color change or a greater color loss between initial and final color measurements.

The following changes in total color ($\Delta E$) are shown below:

| Composition | ΔE |
|---|---|
| 1 | 15.30 |
| 2 | 10.27 |
| 3 | 9.57 |
| Control | 20.08 |

The results above show that the colored hair swatches treated with the inventive compositions statistically significantly (ANOVA test) retained more color (less color loss).

Therefore, the presently claimed composition imparts a color protection to hair.

Example 5: Conditioning effect

The conditioning effect of the inventive composition on the hair was determined by the wet combability test. The ease of combing through wet hair swatches is reflected by the amount of work or total energy needed to pull the comb through the hair. An Instron Tensile Tester Model 4444 was used to measure the total energy required to comb the hair. The more conditioned the hair is, the less energy is required to comb through the hair.

Six (6) hair swatches prepared from normal virgin hair (chemically untreated hair) were cleansed twice with Redken Hair Cleansing Crème for 1 minute and rinsed for 1 minute with water. The total energy required to comb the wet hair was measured.

The hair swatches were then treated with the following composition (quantities being expressed as percentages by weight): laureth-11 carboxylic acid 3.6%, PEG-2 oleamine 2%, mineral oil 1%, and water qs to 100%, using 0.5g of composition / g of hair, for 1 minute and rinsed with water for 10 seconds. The total energy required to comb the wet hair after the treatment was again measured.

The initial energy to comb was 591.05 g-inch and the final energy to comb was 408.79 g-inch.

These data indicate that treated hair required less energy to comb, which is statistically significantly less than the energy required to comb the untreated hair. Therefore, the presently claimed composition imparts a better and more desirable wet combing to hair.

Example 6: Conditioning effect

The test of example 5 was repeated using three (3) hair swatches prepared from normal virgin hair (chemically untreated hair). The swatches were cleansed twice with the Redken Hair Cleansing Crème for 1 minute and rinsed for 1 minute with water. The total energy required to comb the wet hair was measured.

The hair swatches were then treated with the following composition (quantities being expressed as percentages by weight): isostearic acid 1.65%, PEG-15 cocamine 15%, capric/caprylic triglyceride 1%, water qs. to 100% (clear composition), using 0.5g of composition / g of hair, for 1 minute and rinsed with water for 10 seconds. The total energy required to comb the wet hair after the treatment was again measured.

The initial energy to comb was 401.07 g-inch and the final energy to comb was 46.27 g-inch.

These data indicate that treated hair required less energy to comb, which is statistically significantly less than the energy required to comb the untreated hair. Therefore, the presently claimed composition imparts a better and more desirable wet combing to hair.

The foregoing description illustrates and describes the present disclosure. Additionally, the disclosure shows and describes only the preferred embodiments of the disclosure, but, as mentioned above, it is to be understood that it is capable of changes or modifications within the scope of the concept as expressed herein, commensurate with the above teachings and/or skill or knowledge of the relevant art. The embodiments described hereinabove are further intended to explain best modes known of practicing the invention and to enable others skilled in the art to utilize the disclosure in such, or other, embodiments and with the various modification required by the particular applications or uses disclosed herein. Accordingly, the description is not intended to limit the invention to the form disclosed herein. Also, it is intended that the appended claims be construed to include alternative embodiments.

All publications, patents and patent applications cited in this specification are herein incorporated by reference, and for any and all purposes, as if each individual publication, patent or patent application were specifically and individually indicated to be incorporated by reference. In the case of inconsistencies, the present disclosure will prevail.

**Claims**

1. A composition comprising:

    (a) at least one alkoxylated monoamine;

(b) at least one organic acid chosen from alkyl acids, alkoxylated monoacids, and mixtures thereof;
(c) at least one lipophilic compound; and
(d) at least one solvent comprising water.

2. The composition of claim 1, wherein the at least one alkoxylated monoamine is chosen from compounds corresponding to formula (I):

$$RN[(R'CHCH_2O)_xH][(R'CHCH_2O)_yH] \qquad (I)$$

wherein R is a hydrocarbon radical containing at least 6 carbon atoms, preferably from 8 to 30 carbon atoms, more preferably from 12 to 24 carbon atoms;
x and y, independently of one another, represent numbers of from 0 to 100 provided that the sum of x+y is greater than 0;
the groups R', which may be identical or different, represent hydrogen or an alkyl group, such as in particular a methyl group.

3. The composition of claim 1, wherein the at least one alkoxylated monoamine is chosen from compounds corresponding to formula (II):

$$RNR''[(R'CHCH_2O)_xH] \qquad (II)$$

wherein R is a hydrocarbon radical containing at least 6 carbon atoms, preferably from 8 to 30 carbon atoms, more preferably from 12 to 24 carbon atoms;
x represents a number of from 1 to 100;
R' represents hydrogen or an alkyl group, such as in particular a methyl group; and
R'' is hydrogen or a hydrocarbon radical, that may also contain an alkoxylated moiety and/or one or more heteroatoms such as oxygen and nitrogen.

4. The composition of claim 1, wherein the at least one alkoxylated monoamine is chosen from compounds corresponding to formula (III):

$$R(R'CHCH_2O)_x(R'CHCH_2O)_yNH_2 \qquad (III)$$

wherein R is a hydrocarbon radical containing at least 6 carbon atoms, preferably from 8 to 30 carbon atoms, more preferably from 12 to 24 carbon atoms;
x and y, independently of one another, represent numbers of from 0 to 100 with the proviso that the sum of x+y is greater than 0;
the groups R', which may be identical or different, represent hydrogen or an alkyl group, such as in particular a methyl group.

5. The composition of any preceding claim, wherein the at least one alkoxylated monoamine is present in an amount of from 0.1 to 50 % by weight, preferably from 0.5 to 30% by weight, more preferably from 1 to 20% by weight, based on the total weight of the composition.

6. The composition of any preceding claim, comprising at least one alkyl acid chosen from fatty monocarboxylic acids, fatty phosphoric acids and mixtures thereof.

7. The composition of claim 6, wherein the fatty monocarboxylic acids are chosen from compounds of formula (IA):

$$RCOOH \qquad (IA)$$

wherein R is a hydrocarbon radical containing at least 6 carbon atoms, preferably from 8 to 30 carbon atoms, more preferably from 12 to 24 carbon atoms.

8. The composition of claim 6, wherein the fatty phosphoric acids are chosen from compounds of formula (IIA):

$$R\text{-}O\text{-}P(O)(OH)_2 \qquad (IIA)$$

wherein R is a hydrocarbon radical containing at least 6 carbon atoms, preferably from 8 to 30 carbon atoms, more preferably from 12 to 24 carbon atoms.

9. The composition of any preceding claim, comprising at least one alkoxylated monoacid chosen from compounds corresponding to formula (IB):

$$RO[CH_2O]_u[(CH_2)_xCH(R')(CH_2)_yO]_v[CH_2CH_2O]_wCH_2COOH \qquad (IB)$$

wherein:

R is a hydrocarbon radical containing from 6 to 40 carbon atoms, preferably from 8 to 30 carbon atoms, more preferably from 12 to 24 carbon atoms;

u, v and w, independently of one another, represent numbers of from 0 to 60, provided that the sum u + v + w is greater than 0;

x and y, independently of one another, represent numbers of from 0 to 13, where the sum x + y is greater than or equal to 0;

R' represents hydrogen or an alkyl group, and preferably a methyl group.

10. The composition of any preceding claim, wherein the at least one organic acid chosen from alkyl acids, alkoxylated monoacids and mixtures thereof, is present in an amount of from 0.1 to 50% by weight, preferably from 0.5 to 30% by weight, and more preferably 1 to 20% by weight, based on the total weight of the composition.

11. The composition of any preceding claim, wherein the ratio of the amine number of the at least one alkoxylated monoamine to the total acid number of organic acid(s) chosen from alkyl acids, alkoxylated monoacids and mixtures thereof, is from 1:10 to 10:1, preferably from 1:5 to 5:1, and more preferably from 1:2 to 2:1.

12. The composition of any preceding claim, wherein the at least one lipophilic compound is chosen from oils, fatty esters, hydrocarbon oils, silicones, waxes, fatty alcohols, lipophilic vitamins and esters thereof, organic sunscreens, phospholipids, and mixtures thereof, and is preferably present in an amount of from 0.1 to 50% by weight, more preferably from 0.1 to 30% by weight, and even more preferably from 0.5 to 15% by weight, based on the total weight of the composition.

13. The composition of any preceding claim, wherein the solvent is present in an amount from 10 to 95% by weight, preferably from 50 to 85% by weight, and more preferably from 60 to 80% by weight, based on the total weight of the composition, and comprises preferably at least 20% by weight of water, more preferably at least 50% by weight of water, even more preferably at least 80% by weight of water, based on the total weight of the solvent, the solvent further comprising optionally at least one $C_1$-$C_4$ alcohol.

14. A method of making a clear composition involving the steps of:

(a) providing at least one alkoxylated monoamine;
(b) providing at least one organic acid chosen from alkyl acids, alkoxylated monoacids, and mixtures thereof;
(c) providing at least one lipophilic compound;
(d) providing at least one solvent comprising water; and
(e) mixing the compounds as defined in steps (a) to (d) to form a composition that is clear in appearance.

15. A method of cosmetic treatment of a keratinous substrate involving the step of applying onto said keratinous substrate a composition as defined in any one of claims 1 to 13.